Europäisches Patentamt

European Patent Office

Office européen des brevets

(19)

(11) Veröffentlichungsnummer: **0 253 130 B1**

# EUROPÄISCHE PATENTSCHRIFT

(12)

(45) Veröffentlichungstag der Patentschrift: **13.03.91**

(51) Int. Cl.⁵: **A61F 5/00, A61F 13/20**

(21) Anmeldenummer: **87108250.9**

(22) Anmeldetag: **06.06.87**

(54) **Einrichtung zur druckgeminderten Abgabe von Darmgasen.**

(30) Priorität: **15.07.86 AT 1917/86**

(43) Veröffentlichungstag der Anmeldung:
**20.01.88 Patentblatt 88/03**

(45) Bekanntmachung des Hinweises auf die
Patenterteilung:
**13.03.91 Patentblatt 91/11**

(84) Benannte Vertragsstaaten:
**AT BE CH DE ES FR GB GR IT LI LU NL SE**

(56) Entgegenhaltungen:
**DE-A- 1 278 700**       **DE-A- 1 566 659**
**DE-A- 1 642 040**       **DE-A- 2 150 771**
**DE-C- 583 592**         **DE-U- 8 701 908**
**FR-A- 357 772**         **FR-A- 513 639**
**FR-A- 970 225**         **GB-A- 15 173**

(73) Patentinhaber: **Ghedina, Dino**
**Schillerstrasse 4**
**A-6020 Innsbruck(AT)**

(72) Erfinder: **Ghedina, Dino**
**Schillerstrasse 4**
**A-6020 Innsbruck(AT)**

(74) Vertreter: **Torggler, Paul, Dr. et al**
**Wilhelm-Greil-Strasse 16**
**A-6020 Innsbruck(AT)**

Rank Xerox (UK) Business Services

## Beschreibung

Die Erfindung betrifft eine Einrichtung zur druckgeminderten Abgabe von Darmgasen, mit einem eine gerundete, vordere Einführseite aufweisenden, jenseits des Schließmuskels positionierbaren Hohlkörper aus flexiblem Material, dessen Wandung Gaseintrittsöffnungen aufweist und einen Sammelraum für die Darmgase umschließt, sowie mit einem, an den hinteren Endbereich des Hohlkörpers dicht angeschlossenen Röhrchen, dessen den Schließmuskel durchdringender Teil einen geringeren Außendurchmesser als der Hohlkörper aufweist, und dessen die Gasaustrittsöffnung umfassendes äußeres Ende einen Handhabungsteil bildet.

Eine derartige Einrichtung ist beispielsweise der DE-A-2 150 771 zu entnehmen. Das mit dem Hohlkörper verbundene Röhrchen ist mit einem die Einführtiefe begrenzenden Flansch versehen, in dem die Gasaustrittsöffnung vorgesehen ist. Der Außendurchmesser des Röhrchens beträgt mehr als die Hälfte des Außendurchmessers des Hohlkörpers, sodaß der Schließmuskel durch den Hohlkörper dauernd aufgeweitet wird und eine länger anhaltende Verwendung unangenehm ist. Eine Fullung des Hohlkörpers mit Kot, die sich nicht vermeiden läßt, verlegt das Röhrchen und behindert die Gasabführung. Im übrigen besteht die Gefahr, daß der Kot unkontrolliert aus dem Hohlkörper austreten kann, da dieser keinen Sammelraum bildet.

Aus der DE-C-583 592 ist eine weitere derartige Einrichtung bekannt, bei der an einen, seitliche Druckbrechungen in der Wandung und eine gerundete Einführseite aufweisenden Hohlkörper ein gekrümmtes Rohr ansteckbar ist, dessen Durchmesser nur geringfügig unter dem Durchmesser des Sammelraumes liegt, und das am äußeren Ende eine Verdickung aufweist, die die Einführtiefe begrenzt. Ein in die Verdickung einsetzbares Verschlußstück weist eine Gasaustrittsöffnung auf. Da das Rohr sich verlegt, womit der Gasdurchtritt unterbunden ist, ist hiefür ein weiteres Röhrchen zentral eingeordnet, das sich von der Einführseite bis zum Verschlußstück erstreckt, und an den beiden anliegenden Enden seitliche Öffnungen aufweist. Der den Schließmuskel durchdringende Abschnitt des äußeren Rohres weitet diesen in einem beträchtlichen Ausmaß auf und stellt einen für die Abführung der Gase kaum nützbaren Bauteil dar.

Die Erfindung hat es sich nun zur Aufgabe gestellt, eine Einrichtung der eingangs genannten Art zu schaffen, die eine möglichst geringe Beanspruchung des Schließmuskels und wesentlich verbesserte Verwendungseigenschaften aufweist

Erfindungsgemäß wird dies nun dadurch gelöst, daß das Röhrchen als Schlauch ausgebildet ist, der sich ins Innere des Hohlkörpers erstreckt, wobei das innere Ende des Schlauches näher zur gerundeten Einführseite liegt, als die vorderste Gaseintrittsöffnung des Hohlkörpers, daß der Schlauch von durchgehend gleicher Dicke ist, d.h.: dessen Handhabungsteil denselben Außendurchmesser wie der den Schließmuskel und den Sammelraum durchdringende Teil aufweist, wobei der Innendurchmesser des Schlauches so bemessen ist, daß die Darmgase in aufgrund der Verdünnung durch die Außenluft nicht geruchsbelästigenden Mengen austreten, und daß der Schlauch eine die Positionierung des Hohlkörpers erlaubende Länge besitzt.

Der wesentlich geringere Schlauchaußendurchmesser bewirkt eine "Schonung" des Schließmuskels, der nach der Positionierung des Hohlkörpers nur den dünnen Schlauch umspannt, und damit nur minimal aufgeweitet ist. Wie Versuche gezeigt haben, besitzt das nahe der Einführseite liegende innere Ende des Schlauches trotz des ausschließlich im Hohlkörper gegebenen Kotsammelraumes eine ausreichende Gasdurchlässigkeit, die sich erst nach längerem Gebrauch verringert. Tragezeiten von mehreren Stunden können so problemlos erzielt werden. Hiezu trägt auch der Wegfall eines die Einführtiefe begrenzenden Flansches bei.

Das innere Ende des Schlauches kann zusätzlich am Ende abgeschrägt sein oder Umfangsöffnungen aufweisen, um den Eintrittsquerschnitt zu vergrößern.

Der geringere Querschnitt der Gasaustrittsseite erbringt weiters den Vorteil, daß einerseits eine häufige, durch den geringen Druck geräuschlose Abgabe in kleinsten Mengen möglich wird, und andererseits Geruchsbelästigungen entfallen, da die austretenden Kleinstmengen von der Außenluft raschest so weit verdünnt werden, daß praktisch keine Geruchswahrnehmung mehr erfolgt.

Eine bevorzugte Ausführung der Einrichtung kann insbesondere darin bestehen, daß der Hohlkörper aus zwei ineinandergesteckten Teilen zusammengesetzt ist, wobei der vordere Teil die kappenförmige Einführseite und Gaseintrittsöffnungen in der Wandung aufweit, und der hintere Teil mit dem Schlauch versehen ist.

Um die Handhabung der Einrichtung ausschließlich mittels des äußeren Schlauchendes zu ermöglichen, weist der aus flexiblem Material bestehende Schlauch vorzugsweise eine die Positionierung ermöglichende Steifikeit auf, sodaß keine zusätzliche Setzhilfe benötigt wird.

Es ist aber ebenso denkbar, daß der Hohlkörper bzw. dessen hinterer Teil einen Abschnitt mit verringertem Außendurchmesser aufweist, auf den ein entfernbares Positionierröhrchen aufgeschoben ist, dessen Außendurchmesser dem des Hohlkörpers bzw. dessen vorderen Teiles entspricht.

Dieses als Setzhilfe dienende Röhrchen wird nach der Positionierung herausgezogen und weggeworfen, wobei es vorzugsweise ebenso wie die gesamte Einrichtung aus einem verrottbaren Material besteht, das problemlos der Kanalisation anvertraut werden kann. Selbstverständlich sollte der Zerfall der Einrichtung erst nach mehrstündiger Verwendung beginnen.

Nachstehend wird nun die Erfindung an Hand der Figuren der beiliegenden Zeichnungen näher beschrieben, ohne darauf beschränkt zu sein. Diese zeigen Längsschnitte durch vier verschiedene Ausführungsbeispiele der Einrichtung.

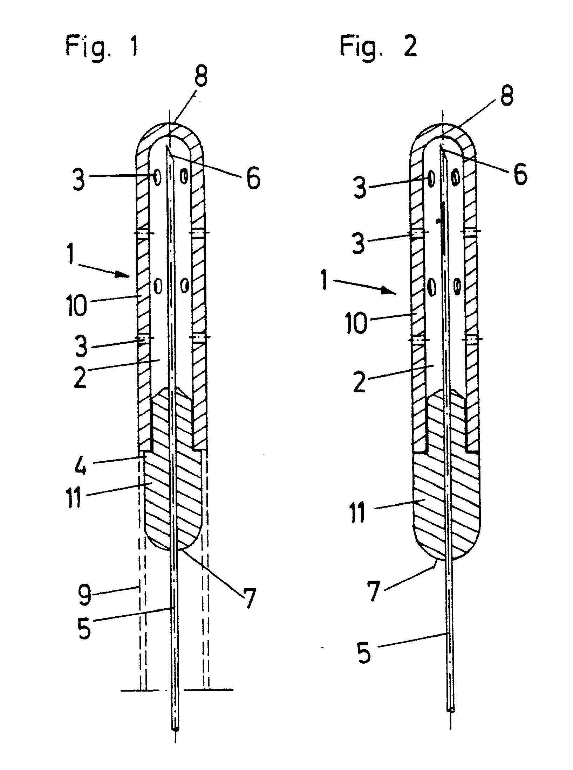

In der Fig. 1 bis 3 ist ein zylindrischer Hohlkörper 1 aus biegsamem Material dargestellt, der auf der Einführseite 8 gerundet ist und einen Sammelraum 2 aufweist. In seiner Wandung sind beliebig verteilte Gaseintrittsöffnungen 3 vorgesehen, durch die die Gase in den Sammelraum 2 gelangen. Diese verlassen den Hohlkörper 1 durch einen Schlauch 5, der an din hinteren Endbereich 7 anschließt. Der Außendurchmesser des Schlauches 5 ist gegenüber dem des Hohlkörpers 1 wesentlich verringert. Der Austrittsquerschnitt ergibt sich durch den Innendurchmesser des Schlauches 5, der beispielsweise zwischen 1 und 2 mm beträgt, während der Durchmesser des Sammelraumes 2 eine Größe von beispielsweise 8 bis 10 mm aufweist. Der Schlauch 5 erstreckt sich im Inneren des Hohlkörpers 1 bis nahe an seine gerundete Einführseite 8 und ist am Ende zur Vergrößerung des Eintrittsquerschnittes mit einer Abschrägung 6 versehen. Diese ist so ausgebildet, daß der elliptische Eintrittsquerschnitt zumindest so groß wie der Querschnitt einer Öffnung 3 ist, um eine frühzeitige Verstopfung des Schlauches 5 durch in den Sammelraum 2 eindringendes Material möglichst hintanzuhalten. Das Ende des Schlauches 5 liegt vorzugsweise näher zur Einführseite 8 als die vordersten Gaseintrittsöffnungen 3. Der Schlauch 5 weist eine Länge auf, die die Positionierung des Hohlkörpers 1 im Raum jenseits des Schließmuskels gestattet, sodaß dieser nur von dem dünnen Schlauch 5 durchdrungen wird.

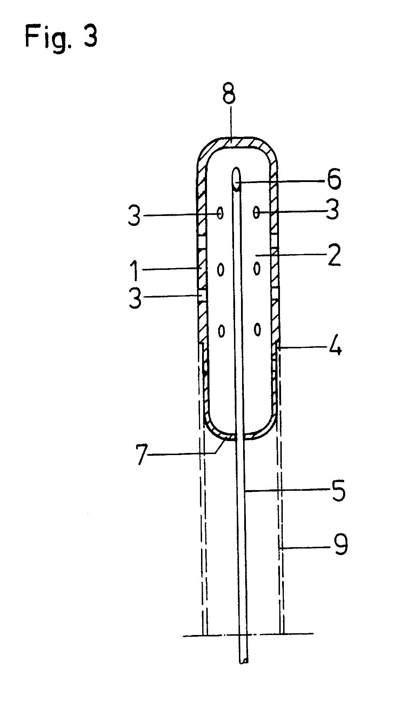

Gemäß den Figuren 1 und 2 ist der Hohlkörper 1 aus zwei ineinandergesteckten Teilen 10, 11 zusammengesetzt. Der vordere Teil 10 umschließt den Sammelraum 2 und enthält die Öffnungen 3. Der hintere Teil 11 dient als Verschlußstopfen und enthält den in den Sammelraum 2 ragenden Schlauch 5. In Fig. 3 ist ein einteiliger Hohlkörper 1 gezeigt. Die Biegefestigkeit des Schlauches 5 ist bevorzugt so groß, daß er als Setzhilfe verwendet werden kann. Der Hohlkörper 1 besitzt in diesem Fall gemäß Fig. 2 einen einheitlichen Außendurchmesser. Wie aus den Fig. 1 und 3 ersichtlich, kann als Setzhilfe ein strichliert angedeutetes Positionierröhrchen 9 verwendet werden, das nach dem Einführen in den Darm abgezogen wird. Nach dieser Ausführung wird der wünschenswerte einheitliche Außendurchmesser auf Grund eines um die Wandstärke des Positionierröhrchens 9 verkleinerten Abschnittes 4 des hinteren Teiles 11 (Fig. 1) bzw. des Hohlkörpers 1 (Fig. 3) erreicht, wobei auf den Abschnitt 4 das Positionierröhrchen 8 aufgesteckt ist.

## Ansprüche

1. Einrichtung zur druckgominderten Abgabe von Darmgasen, mit einem eine gerundete, vordere Einführseite (8) aufwiesenden, jenseits des Schließmuskels positionierbaren Hohlkörper (1) aus flexiblem Material, dessen Wandung Gaseintrittsöffnungen (3) aufweist und einen Sammelraum (2) für die Darmgase umschließt, sowie mit einem, an den hinteren Endbereich (7) des Hohlkörpers (1) dicht angeschlossenen Röhrchen, dessen den Schließmuskel durchdringender Teil einen geringeren Außendurchmesser als der Hohlkörper (1) aufweist, und dessen die Gasaustrittsöffnung umfassendes äußeres Ende einen Handhabungsteil bildet, dadurch gekennzeichnet, daß das Röhrchen als Schlauch (5) ausgebildet ist, der sich ins Innere des Hohlkörpers (1) erstreckt, wobei das innere Ende des Schlauches (5) näher zur gerundeten Einführseite (8) liegt, als die vorderste Gaseintrittsöffnung (3) des Hohlkörpers (1), daß der Schlauch (5) von durchgehend gleicher Dicke ist, d.h. dessen Handhabungsteil denselben Außendurchmesser wie der den Schließmuskel und den Sammelraum (2) durchdringenden Teil aufweist, wobei der Innendurchmesser des Schlauches (5) so bemessen ist, daß die Darmgase in aufgrund der Verdünnung durch die Außenluft nicht geruchsbelästigenden Mengen austreten, und daß der Schlauch (5) eine die Positionierung des Hohlkörpers (1) erlaubende Länge besitzt.

2. Einrichtung nach Anspruch 1, dadurch gekennzeichnet, daß der Hohlkörper (1) aus zwei ineinandergesteckten Teilen (10, 11) zusammengesetzt ist, wobei der vordere Teil (10) die gerundete Einführseite (8) und die Gaseintrittsöffnungen (3) in der Wandung aufweist, und der hintere Teil (11) vom Schlauch (5) durchsetzt ist.

3. Einrichtung nach Anspruch 1 oder 2, dadurch gekennzeichnet, daß der Hohlkörper (1) einen Innendurchmesser von 8 bis 10 mm und der Schlauch (5) einen Innendurchmesser von 1 bis 1,5 mm aufweist.

4. Einrichtung nach einem der Ansprüche 1 bis 3, dadurch gekennzeichnet, daß der Schlauch (5) eine die Positionierung ermöglichende Steifigkeit aufweist.

5. Einrichtung nach Anspruch 1 bis 3, dadurch gekennzeichnet, daß der Hohlkörper (1) bzw. dessen hinterer Teil (11) einen Abschnitt (4) mit verringertem Außendurchmesser aufweist, auf den ein entfernbares Positionierröhren (9) aufgeschoben ist, dessen Außendurchmesser dem des Hohlkörpers (1) bzw. dessen vorderen Teiles (10) entspricht.

6. Einrichtung nach einem der Ansprüche 1 bis 5, dadurch gekennzeichnet, daß sie aus einem verrottbaren Material besteht.

## Claims

1. A device for the discharge of intestinal gases at reduced pressure, comprising a hollow body (1) of flexible material, which has a rounded front insertion end (8) and which can be positioned beyond the sphincter and the wall of which has gas intake openings (3) and a collecting space (2) for the intestinal gases, and comprising a tube which is sealingly connected to the rear end region (7) of the hollow body (1) and the part of which that passes through the sphincter is of a smaller outside diameter than the hollow body (1) and the outer end of which that includes the gas outlet opening forms a handling portion, characterised in that the tube is in the form of a hose (5) which extends into the interior of the hollow body (1), wherein the inner end of the hose (5) is closer to the rounded insertion end (8) than the foremost gas intake opening (3) of the hollow body (1), that the hose (5) is of the same thickness throughout, that is to say the handling portion thereof is of the same outside diameter as the part which passes through the sphincter and the collecting space (2), wherein the inside diameter of the hose (5) is such that the intestinal gases issue in amounts which do not constitute a smell nuisance, by virtue of dilution by the outside air, and that the hose (5) is of a length which permits positioning of the hollow body (1).

2. A device according to claim 1 characterised in that the hollow body (1) comprises two parts (10, 11) which are fitted one into the other, wherein the front part (10) has the rounded insertion end (8) and the gas intake openings (3) in the wall and the rear part (11) has the hose (5) passing therethrough.

3. A device according to claim 1 or claim 2 characterised in that the hollow body is of an inside diameter of from 8 to 10 mm and the hose (5) is of an inside diameter of from 1 to 1.5 mm.

4. A device according to one of claims 1 to 3 characterised in that the hose (5) presents a stiffness which permits positioning.

5. A device according to claim 1 to 3 characterised in that the hollow body (1) or the rear part (11) thereof has a portion (4) of reduced outside diameter, on to which is pushed a removable positioning tube (9), the outside diameter of which corresponds to that of the hollow body (1) or the front part (10) thereof.

6. A device according to one of claims 1 to 5 characterized in that it comprises a degradable material.

## Revendications

1. Dispositif pour diminuer la pression d'émission de gaz intestinaux, comportant un corps creux (1) en matériau flexible pourvu d'un côté d'introduction avant arrondi (8), apte à être positionné audelà du sphincter et dont la paroi possède des orifices d'entrée de gaz (3) et une chambre collectrice (2) pour les gaz intestinaux, et un tube, relié de façon étanche à la zone d'extrémité arrière (7) du corps creux (1), dont la partie traversant le sphincter présente un diamètre extérieur inférieur à celui du corps creux (1) et dont l'extrémité extérieure comportant l'orifice d'évacuation de gaz définit un élément de manipulation, caractérisé en ce que le tube est conçu comme un tuyau (5) qui s'étend jusqu'à l'intérieur du corps creux (1), l'extrémité intérieure du tuyau (5) se trouvant plus près du côté d'introduction arrondi (8) que l'orifice d'entrée de gaz (3) le plus en avant du corps creux (1), en ce que le tuyau (5) présente la même épaisseur sur toute sa longueur, c'est-à-dire que son élément de manipulation présente le même diamètre extérieur que les parties traversant le sphincter et la chambre collectrice (2), le diamètre intérieur du tuyau (5) étant dimensionné pour laisser échapper des quantités de gaz intestinaux qui ne soient pas gênantes par leur odeur, grâce à leur dilution dans l'air extérieur, et en ce que le tuyau (5) possède une longueur permettant le positionnement du corps creux (1).

2. Dispositif selon la revendication 1, caractérisé en ce que le corps creux (1) se compose de deux parties (10, 11) emboîtées l'une dans l'autre, la partie avant (10) comportant le côté d'introduction arrondi (8) et les orifices d'entrée de gaz (3) ménagés dans la paroi, et la partie arrière (11) étant traversée par le tuyau (5).

3. Dispositif selon la revendication 1 ou 2, caractérisé en ce que le corps creux (1) présente un diamètre intérieur de 8 à 10 mm et le tuyau (5) un diamètre intérieur de 1 à 1,5 mm

4. Dispositif selon l'une des revendications 1 à 3, caractérisé en ce que le tuyau (5) offre une rigidité permettant le positionnement du corps creux (1).

5. Dispositif selon les revendications 1 à 3, caractérisé en ce que le corps creux (1), respectivement sa partie arrière (11) présente une section (4) de diamètre extérieur réduit sur laquelle est enfilé un tube de positionnement (9) amovible d'un diamètre extérieur correspondant à celui du corps creux (1), respectivement de la partie avant (10) de celui-ci.

6. Dispositif selon l'une des revendications 1 à 5, caractérisé en ce qu'il est formé d'un matériau apte à pourrir.

Fig. 1

Fig. 2

Fig. 3